# EUROPEAN PATENT APPLICATION

(11) **EP 4 740 978 A1**
(43) Date of publication of application: **13.05.2026**
(21) Application number: 24212251.3
(22) Date of filing: 12.11.2024
(51) Int. Cl.: A61M 5/00

(54) **A PHARMACEUTICAL KIT COMPRISING AN ENCLOSURE WITH SEPARATING MEANS**

(71) Applicant: B. Braun Melsungen AG, 34212 Melsungen (DE)
(72) Inventor: MEDICO, Leonard, 34212 Melsungen (DE); FRAUCHIGER, Daniel, 34212 Melsungen (DE); VALLOTTON, Raphael, 34212 Melsungen (DE)
(74) Representative: Maiwald GmbH

(57) **Abstract**

A pharmaceutical kit, wherein the pharmaceutical kit comprises
- a container comprising a pharmaceutical compound,
- a medical device suitable for receiving the pharmaceutical compound from the container,
- an enclosure enclosing the container and the medical device,

wherein the enclosure comprises at least one wall enclosing a main cavity,
wherein the main cavity is a closed cavity,
wherein the cavity comprises the container and the medical device,
wherein the cavity comprises a separating means separating the main cavity into a container cavity comprising the container and a device cavity comprising the medical device,
wherein the container cavity and the device cavity each are closed cavities, and wherein the enclosure comprises an opening means.

## Description

### Technical Field of the Invention

The present invention is related to packaging pharmaceutical products, in particular packaging pharmaceutical kits. The present invention is further related to the use of such pharmaceutical kits.

### Background of the Invention

In the technical field of packaging, in particular in the technical field of pharmaceutical product, several products are often packaged together in one container. Such packages are usually described as kits. Well-known kits in the field of pharmaceutical products are combination drug kits, diagnostic kits, injectable kits, surgical and wound care kits, antidote and emergency kits, and respiratory treatment kits.

Usually, packaging of such pharmaceutical kits requires sterile packaging. To reduce complexity during handling, these pharmaceutical kits are often already positioned in the container before sterilization. Hence, they are sterilized within the container after packaging.

Usually, sterilization of such pharmaceutical kits is carried out using UV radiation sterilization or ethylene oxide sterilization. Reason is that the lack of humidity present in the packaging container prevents sterilization by heat to be achieved into device cavities, as usually water molecules in the surrounding atmosphere transport the heat. However, the packaging usually contains measures to reduce the water content in the packaging. Thus, in summary, typical pharmaceutical kits require complex sterilization methods.

Additionally, usually, in pharmaceutical kits known from the prior art the products group therein and packaged thereby are put all together in one chamber. For example, patent application EP 0 240 144 A1 discloses a syringe being provided together with a pharmaceutical container in a packaging container within one chamber. Moreover, patent application EP 2 903 660 A1 discloses a prefilled syringe being stored with its plunger and an oxygen absorber in one chamber in a packaging container.

### Summary of the Invention

The solutions provided by the prior art have the disadvantage that the one chamber forms a large headspace. Pharmaceutical containers containing, e.g., an oxygen-sensitive solution, are generally provided in an oxygen-permeable primary container, which is wrapped in a secondary packing with barrier functions. For the sterilization and for the storage of such solutions it is, however, advisable to have a reduced headspace between the primary and the secondary packaging.

Moreover, the solutions known from the prior art have the further disadvantage of potential migration of extractables and leachables from one product to another within the one chamber of the packaging. Generally, it is important to protect pharmaceutical solutions from migrating chemical ingredients, such as volatile, semi-volatile, as well as non-volatile compounds of various nature during the sterilization step, wherein they are exposed to elevated temperature such as in an autoclave, or during shelf-life. Such migrating chemical ingredients which could affect the stability and the quality of the packaged pharmaceutical solution. However, these chemical ingredients may origin from the label, the ink, the stopper of a syringe, etc.

Hence, it is an object of the present invention to provide a pharmaceutical packaging, which enables packaging more than one pharmaceutical product and reducing the secondary headspace of the packaging at the same time.

It is another object of the present invention to provide a pharmaceutical packaging, which enables packaging more than one pharmaceutical product and reducing migration of chemical compounds between the pharmaceutical products at the same time.

It has now surprisingly found out that above-mentioned objects can be achieved by a pharmaceutical kit, wherein the pharmaceutical kit comprises
- a container comprising a pharmaceutical compound,
- a medical device suitable for receiving the pharmaceutical compound from the container,
- an enclosure enclosing the container and the medical device,

wherein the enclosure comprises at least one wall enclosing a main cavity,
wherein the main cavity is a closed cavity,
wherein the cavity comprises the container and the medical device,
wherein the cavity comprises a separating means separating the main cavity into a container cavity comprising the container and a device cavity comprising the medical device,
wherein the container cavity and the device cavity each are closed cavities, and
wherein the enclosure comprises an opening means.

It has been further surprisingly found that above-mentioned object can be achieved by the use of a pharmaceutical kit according to the present invention for sterile storing the pharmaceutical compound.

Moreover, it has been surprisingly found that above-mentioned object can be achieved by the use of a pharmaceutical kit according to the present invention for transferring the pharmaceutical compound to the medical device.

Finally, it has been surprisingly found out that above-mentioned object can be achieved by a process of filling the medical device of the pharmaceutical kit according to the present invention, the process comprising the steps of:
- opening the enclosure;
- opening the container;
- transferring the pharmaceutical compound to the medical device.

The present invention has the advantage that by separation of the container and the medical device, migration of chemical compounds between them is reduced. Furthermore, the secondary headspace is significantly reduced.

### Definitions

The term *'polymer'* as used herein denotes an organic polymer, preferably a polymer selected from polyolefins, polyethylene terephthalate, polystyrene, polyvinyl chloride, or mixtures thereof, more preferably a polyolefin selected from the list consisting of polyethylene, polypropylene, or mixtures thereof.

The term *'liquid-tight'* as used herein denotes a quality of an object to function as a barrier for a liquid, preferably for a water-based liquid.

The term *'barrier layer'* as used herein denotes a material, which can slow down the process of oxygen permeation. Preferably, it comprises, more preferably consists of, a based barrier material, most preferably ethylene-vinyl alcohol (EVOH), or a metal oxide material, most preferably a silicon oxide layer or an aluminum oxide layer. Such barrier layer can require a supporting or binding layer to adhere thereto. Metal oxide-based layers can be deposited directly onto the polymer material (e.g., by chemical vapor deposition).

The term *'heat sealing'* as used herein denotes thermal welding of polymer films. In this process, at least two polymer films are heated until at least the glass transition temperature is reached and the plastics in these areas begin to become liquid or at least low viscous. By simultaneous or subsequent mechanical pressing of these areas, the polymers are mixed together at this point and, after cooling and solidification, form a fixed joint called a seam.

The term *'active pharmaceutical ingredient (API)'* as used herein denotes a substance preferably provided in a pharmaceutical product that is biologically active and responsible for producing intended therapeutic effects. APIs are the key ingredients in medications that treat, diagnose, or prevent diseases. For example, in a pain relief medication like ibuprofen, ibuprofen itself is the API, while other components in the formulation (excipients) may help with the drug's stability, absorption, or delivery. APIs can be small molecules or large molecules such as proteins or antibodies.

The term *'additive'* as used herein denotes further components, which can be present in polymer compositions to modify their physical properties. Examples of additives are antioxidant(s), stabilizer(s), such as process stabilizers and UV stabilizers, acid scavenger(s), metal deactivators, crosslinking agents, such as free radical generating agent(s), e.g., organic peroxide(s), scorch retarder(s), crosslinking booster(s), processing aid(s), flame retardant additive(s), water tree retardant additive(s), inorganic filler(s), and voltage stabilizer(s). These groups of additives and the individual additive compounds therein are usually well known in the polymer field.

The term *'sterile'* as used denotes the status of an object having a significantly reduced number of bacteria and/or viruses on its surface to reduce the risk of an infection. In particular, the term *'sterile'* denotes an object or substance, which has a bioburden load of lower than 10⁻⁶. The bioburden load can be measured i.e., according to ISO 11737-1:2018.

The term *'sterilization'* as used herein denotes a method to destroy all forms of living microorganisms from a substance. As there is always a certain probability of at least one microorganism to survive such procedure, the aim of sterilization is the reduction of initially present microorganisms or other potential pathogens. Generally, sterilization is accepted to be achieved if the bioburden load of the substance of object to be sterilized is lower than 10⁻⁶. The bioburden load can be measured i.e., according to ISO 11737-1:2018. Sterilization can be achieved using several methods. In one sterilization process the object is heated up to at least 105 °C to achieve a sterile object. Thereby, the object should not be deformed by the elevated temperature. Preferably, the heating step is performed in an autoclave. In another sterilization process, the object is brought into contact with toxic gases such as a mixture of ethylene oxide and carbon dioxide. Filtration methods are also used to sterilize liquids, i.e., by using membrane filters, Seitz filters, and/or candle filters. Finally, sterilization can be achieved by indirect energy import into or onto the object, e.g., by ultrasonic waves, ultraviolet light, as well as by high energy particles (such as electrons, gamma- or X-rays).

The term *'seam'* as used herein denotes an area of at least two connected walls including at least one edge of each wall, of an enclosure, preferably bag, at which the two walls are connected, i.e., by gluing or welding, thereby forming a seam area, a seam edge, and an inner boundary of the edge.

The term *'seam width'* as used herein denotes the distance between the seam edge and the inner boundary of the seam in a direction perpendicular to the seam edge. Usually, the seam width is substantially homogeneous, i.e., does not vary in the direction of the seam edge. However, variation of the seam width can be part of the overall design of the enclosure.

The term *'oxygen depleted gas'* denotes a gas, which has a lower concentration of oxygen than air, preferably no or only trace amounts of oxygen. Hence, preferably, the oxygen depleted gas comprises, preferably consists of, a gas selected from nitrogen, carbon dioxide, a noble gas, or mixtures thereof. Most preferably, the oxygen depleted gas comprises, preferably consists of, nitrogen.

The term *'secondary headspace'* as used herein denote the volume between an object packaged by an enclosure and the enclosure itself. It is called secondary headspace, as the object often is a container comprising a compound, preferably a liquid compound, and another headspace fill with gas, which is usually denoted as primary headspace. Preferably, the secondary headspace is filled with an oxygen depleted gas and/or a water depleted gas.

### Detailed Description of the Invention

As set out above the present invention is related to a pharmaceutical kit, a process for preparing such a pharmaceutical kit, and uses of the pharmaceutical kit. These will now be explained in detail.

### Pharmaceutical kit of the Invention

A pharmaceutical kit, wherein the pharmaceutical kit comprises
- a container comprising a pharmaceutical compound,
- a medical device suitable for receiving the pharmaceutical compound from the container,
- an enclosure enclosing the container and the medical device,

wherein the enclosure comprises at least one wall enclosing a main cavity,
wherein the main cavity is a closed cavity,
wherein the cavity comprises the container and the medical device,
wherein the cavity comprises a separating means separating the main cavity into a container cavity comprising the container and a device cavity comprising the medical device,
wherein the container cavity and the device cavity each are closed cavities, and
wherein the enclosure comprises an opening means.

The containers comprised by the pharmaceutical kit of the present invention are preferably selected from blister packs, vials, bottles, sachets, ampoules, tubes, cartridges and pens, and inhalers. Blister packs are usually used for tablets and capsules, offering individual protection. Vials are usually glass or plastic containers for injectable medications, often sealed with rubber stoppers. Bottles are usually plastic or glass bottles for liquids, syrups, or pills. Sachets are usually single-dose packets for powders, granules, or gels. Ampoules are usually small, sealed glass containers for single-dose liquid medications. Tubes are usually used for ointments, creams, and gels. Cartridges and pens are usually used for injectables such as insulin in reusable delivery systems. Inhalers are devices for delivering respiratory medications. Preferably, the containers comprised by the pharmaceutical kit of the present invention are containers suitable for comprising a liquid. Hence, preferably, the container comprised in the pharmaceutical kit of the present invention is selected from the list consisting of vials, bottles, and ampoules.

The pharmaceutical compound as comprised in the container comprised in the pharmaceutical kit of the present invention can be selected from a solid or a liquid compound. If the pharmaceutical compound is a solid compound, it is preferably a pulverized solid compound. Preferably, the pharmaceutical compound is present in liquid form, preferably in solution, more preferably in aqueous solution.

The pharmaceutical compound can be a compound as usually used in pharmaceutical applications, e.g. sodium chloride. However, more specifically, the pharmaceutical compound could also be an active pharmaceutical ingredient, preferably an active pharmaceutical ingredient in solution. It should be understood that in specific cases, the active pharmaceutical ingredient is sensitive to specific environmental impacts, i.e. UV or VIS radiation, oxygen, or water.

The enclosure of the pharmaceutical kit of the present invention is preferably selected from the list consisting of an overwrap, a pouch, a flow-wrap, and rigid or semi-rigid blisters. Hence, preferably, the wall of the enclosure of the pharmaceutical kit of the present invention comprises, preferably consists of, at least one polymer film, preferably a flexible, thermoformable, and/or thermoformed polymer film.

Usually, overwraps are formed by wrapping the polymer film around the product and heat sealing it at its edges. Overwraps might also be formed by more than one polymer film. A flow-wrap is similar to an overwrap but can be produced in continuous mode. Blisters are usually formed by thermoforming a plastic cavity from a thermoformable polymer film and covering the plastic cavity with a flexible polymer film.

Preferably, the separating means comprises, preferably consists of, the same material as the enclosure. Hence, more preferably, the separating means comprises, preferably consists of, the at least one polymer film. The separating means are used for separating the main cavity into a container cavity comprising the container and a device cavity comprising the medical device. Preferably, the separating means comprises, more preferably consists of, a peelable connection of the at least one polymer film. This embodiment has the advantage that the separating means can be provide with low effort and costs. If the separating means comprises the peelable connection, the separating means could also include a polymer film as a wall separating the two cavities. If the separating means consists of the peelable connection, the chambers are only connected via the seam, not a polymer wall (i.e. the main cavity has been heat sealed along a line forming the separating means).

The opening can be selected from notches, zippers, or other means. Preferably, however, the opening means comprises, preferably consists of, a peelable connection of the at least one polymer film.

The at least one polymer film is preferably a sealable polymer film. Furthermore, the polymer film is preferably a liquid-tight film.

In a preferred embodiment of the present invention, the enclosure is made from a polymer film, wherein the polymer film comprises, preferably consists of, a polymer material. The polymer material is preferably selected from the list consisting of polyethylene, polypropylene, polyethylene terephthalate, polyamide, ethylene vinyl alcohol, or mixture thereof. More preferably the polymer material is selected from polyethylene or polypropylene. These materials have an ideal balance of material properties, processability, inertia in view of the neuraxial drug, and low price. Additionally, the film can preferably comprise one or more additives.

Preferably, the polymer film has a haze value measured according to ASTM D1003, Procedure B, of less than 60%, preferably less than 30%, more preferably less than 25%, and most preferably less than 15%. This ensures that the user can see the products comprised by the enclosure.

Preferably, the polymer material of the polymer film has a glass transition temperature *T_{g}* measured according to ASTM D3418, of not more than 80 °C, preferably not more than 60 °C, more preferably not more than 40 °C, and most preferably not more than 20 °C. This ensures thermal stability in particular in view of the sterilization conditions used.

In a preferred embodiment, the polymer film comprises, preferably consists of, a multilayer polymer material. The multilayer polymer material can be prepared by coextrusion, e.g., in a blown-extrusion process, in cast-extrusion process, or in an extrusion-laminating process, optionally after deposition, preferably chemical vapor deposition, of a barrier layer onto one of the films. Hence, the polymer film of the wall of the enclosure can have an innermost layer, wherein the innermost layer denotes the layer forming at least parts, preferably all of, the inner surface of the enclosure, and the polymer film of the wall of the enclosure can have an outermost layer, wherein the outermost layer denotes the layer forming at least parts, preferably all of, the outer surface of the enclosure.

The innermost layer usually has the function of providing inertia of the polymer film of the wall of enclosure the in view of packaged products, i.e. the container and the medical device. Hence, preferably, the material of the innermost layer is a material, which prevents or at least reduces migration of compounds from the multilayer polymer material into the enclosure. Furthermore, the material of the innermost layer is preferably a material which prevents or at least reduces migration of compounds from the packaged products into the multilayer polymer material of the enclosure. Particularly preferably, the material of the innermost layer is a material which prevents or at least reduces migration of the pharmaceutical compound into the multilayer polymer material of the enclosure. Hence, most preferably, the material of the innermost layer of the enclosure according to the present invention is selected from the list consisting of a polypropylene, a polyethylene, or a cyclic olefin, more preferably is a cyclic olefin.

The function of the outermost layer of the multilayer the polymer film of the wall of enclosure is to provide impact protection from the environment to protect the wall of the enclosure. Hence, preferably, the material of the outermost layer is preferably a polymer material having improved mechanical properties, such as tensile strength, impact strength, and/or toughness. Hence, preferably, the material of the outermost layer of the wall of the enclosure is a polyolefin material, most preferably is selected from a polyethylene or a polypropylene.

The multilayer polymer material of the wall of the enclosure according to the present invention preferably further comprises a barrier layer. The barrier layer preferably comprises, more preferably consists of, a material selected from ethylene vinyl alcohol (EVOH) or a metal oxide, preferably aluminum oxide or silicon oxide. It should be noted that a barrier layer made from EVOH usually does not need a support layer, as it is extruded.

In a preferred embodiment of the present invention, the peelable connection of the separating means is a sealed connection, preferably a sealed connection of the at least one polymer film, most preferably a sealed seam of the at least one polymer film. Such a sealed seam can be easily produced by heat sealing two polymer films together. Preferably, the peelable connection of the separating means has a sealing strength according to ASTM F88-94 in the range from 0.15 to 0.7 N/mm, preferably from 0.15 to 0.5 N/mm, and most preferably from 0.15 to 0.35 N/mm. This ensures that the separating means can be easily opened.

Likewise, preferably, the peelable connection of the opening means is a sealed connection, preferably a sealed connection of the at least one polymer film, most preferably a sealed seam of the at least one polymer film. Preferably, the peelable connection of the opening means has a sealing strength according to ASTM F88-94 in the range from 0.15 to 0.7 N/mm, preferably from 0.15 to 0.5 N/mm, and most preferably from 0.15 to 0.35 N/mm. This ensures that the enclosure can be easily opened.

The sealed seam of the opening means preferably extends circumferentially around the enclosure. This allows opening the whole enclosure by opening the sealed seam of the opening means.

Likewise, the sealed seam of the opening means can be in connection with the sealed seam of the separating means. More preferably, the sealed seam of the opening means is at at least two positions in connection with the sealed seam of the separating means. Combining the sealed seams of the opening means and the separating means enables opening of the whole pharmaceutical kit at once by peeling the polymer film from the seal.

In the pharmaceutical kit according to the present invention, the container comprised in the enclosure defines a primary headspace, wherein the enclosure and the container cavity define a secondary headspace. Preferably, the primary headspace and/or the secondary headspace is filled with a gas, preferably with an inert gas, more preferably with a gas selected from nitrogen, noble gases, or oxygen depleted gases. This assures that oxygen sensitive pharmaceutical compounds are protected.

The secondary headspace can optionally comprise an oxygen absorber to maintain the low oxygen content in the pharmaceutical kit.

Preferably, the pharmaceutical kit is sterile, more preferably has been sterilized by heating the pharmaceutical kit up to at least 105 °C, and most preferably up to at least 121 °C.

The medical device is preferably selected from the list consisting of syringes, preferably pre-filled syringes, conventional syringes, or auto-injectors; pumps; topical applicators, preferably transdermal patches, gel applicators, wipes, and ointment/cream dispensers; oral delivery devices, preferably oral dispensers, and capsule/tablet dispensers; injection pens, preferably insulin pens, and multi-dose pens; catheters; needle-free injectors; micro-needles; and infusion sets; infusion transfer devices; adaptors; connectors. More preferably, the medical device can be connected to the container. Most preferably, the medical device is a syringe.

Preferably, the medical device comprises means for transferring the pharmaceutical compound into the medical device, such as connectors, tubes, syringe tips or needles, and valves.

### Uses of the pharmaceutical kit

The present invention is further concerned with the use of a pharmaceutical kit according to the present invention for sterile storing the pharmaceutical compound. It is in particular preferred that during storing also oxygen-, water- or light-sensitive pharmaceutical compounds are not degraded.

Furthermore, the present invention is concerned with the use of a pharmaceutical kit according to the present invention for filling the pharmaceutical compound into the medical device. Thereby the present invention in particular enables the packaging of medical devices, which comprise chemical compounds which tend to migrate and might migrate into the container if no separating means were present in the pharmaceutical kit.

### Process for transferring the pharmaceutical compound

Finally, the present invention relates to the process of transferring the pharmaceutical compound to the medical device of the pharmaceutical kit according to the present invention, the process comprising the steps of:
- opening the enclosure;
- opening the container;
- transferring the pharmaceutical compound to the medical device.

In a preferred embodiment of the process of the invention, the step of opening the enclosure comprises peeling the polymer film from the sealed seam of the opening means.

## Claims

1. A pharmaceutical kit, wherein the pharmaceutical kit comprises
- a container comprising a pharmaceutical compound,
- a medical device suitable for receiving the pharmaceutical compound from the container,
- an enclosure enclosing the container and the medical device,
wherein the enclosure comprises at least one wall enclosing a main cavity,
wherein the main cavity is a closed cavity,
wherein the cavity comprises the container and the medical device,
wherein the cavity comprises a separating means separating the main cavity into a container cavity comprising the container and a device cavity comprising the medical device,
wherein the container cavity and the device cavity each are closed cavities, and wherein the enclosure comprises an opening means.

2. The pharmaceutical kit according to claim 1, wherein the wall comprises, preferably consists of, at least one polymer film, preferably a flexible, thermoformable, and/or thermoformed polymer film.

3. The pharmaceutical kit according to any of the preceding claims 1 to 2, wherein the separating means comprises, preferably consists of, the polymer film.

4. The pharmaceutical kit according to claim 3, wherein the separating means comprises, preferably consists of, a peelable connection of the at least one polymer film.

5. The pharmaceutical kit according to any of the preceding claims 1 to 4, wherein the opening means comprises, preferably consists of, a peelable connection of the at least one polymer film.

6. The pharmaceutical kit according to any of the preceding claims 2 to 5, wherein the at least one polymer film is a weldable polymer film.

7. The pharmaceutical kit according to any of the preceding claim 2 to 6, wherein the polymer film is a multilayer polymer film.

8. The pharmaceutical kit according to claim 7, wherein the multilayer polymer film comprises a support layer and/or a barrier layer.

9. The pharmaceutical kit according to claim 8, wherein the barrier layer comprises, preferably consists of, a material selected from ethylene vinyl alcohol (EVOH) or a metal oxide, preferably aluminum oxide or silicon oxide.

10. The pharmaceutical kit according to any of the preceding claims 1 to 9, wherein the pharmaceutical compound is present in liquid form, preferably in solution, more preferably in aqueous solution.

11. The pharmaceutical kit according to any of the preceding claims 1 to 10, wherein the medical device is selected from the list consisting of syringes, preferably pre-filled syringes, conventional syringes, or auto-injectors; pumps; topical applicators, preferably transdermal patches, gel applicators, wipes, and ointment/cream dispensers; oral delivery devices, preferably oral dispensers, and capsule/tablet dispensers; injection pens, preferably insulin pens, and multi-dose pens; catheters; needle-free injectors; micro-needles; and infusion sets; infusion transfer devices; adaptors; connectors.

12. Use of a pharmaceutical kit according to any of the preceding claims 1 to 11 for sterile storing the pharmaceutical compound.

13. Use of a pharmaceutical kit according to any of the preceding claims 1 to 11 for filling the pharmaceutical compound into the medical device.

14. Process of filling the medical device of the pharmaceutical kit according to any of the preceding claims 1 to 11, comprising the steps of:
- opening the enclosure;
- opening the container;
- transferring the pharmaceutical compound to the medical device.

15. Process according to claim 14, wherein the step of opening the enclosure comprises peeling the polymer film from the sealed seam of the opening means,.
